(19) Europäisches Patentamt / European Patent Office / Office européen des brevets

(11) **EP 4 197 433 B1**

(12) **FASCICULE DE BREVET EUROPEEN**

(45) Date de publication et mention
de la délivrance du brevet:
**05.02.2025 Bulletin 2025/06**

(21) Numéro de dépôt: **22214276.2**

(22) Date de dépôt: **16.12.2022**

(51) Classification Internationale des Brevets (IPC):
*A61B 5/00* (2006.01)  *A61B 5/1455* (2006.01)
*G01N 21/47* (2006.01)  *G01B 9/02* (2022.01)
*G01S 17/88* (2006.01)  *G01S 17/90* (2020.01)
*G01N 21/17* (2006.01)  *G01N 21/49* (2006.01)
*G02F 1/29* (2006.01)

(52) Classification Coopérative des Brevets (CPC):
**A61B 5/0075; A61B 5/1455; G01N 21/31;
G01N 21/474; G01N 21/49; G01S 7/4917;
G01S 7/497; G01S 17/88;** G02F 1/29

(54) **DISPOSITIF ET PROCÉDÉ DE FOCALISATION D'UN RAYONNEMENT LUMINEUX COHÉRENT DANS UN MILIEU DIFFUSANT**

VORRICHTUNG UND VERFAHREN ZUR FOKUSSIERUNG KOHÄRENTER LICHTSTRAHLUNG IN EINEM STREUENDEN MEDIUM

DEVICE AND METHOD FOR FOCUSING COHERENT LIGHT RADIATION IN A SCATTERING MEDIUM

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priorité: **17.12.2021 FR 2113799**

(43) Date de publication de la demande:
**21.06.2023 Bulletin 2023/25**

(73) Titulaire: **Commissariat à l'Energie Atomique et
aux Energies
Alternatives
75015 Paris (FR)**

(72) Inventeurs:
• **KOENIG, Anne
38054 Grenoble cedex 09 (FR)**
• **ANDRE, Luc
38054 Grenoble cedex 09 (FR)**

(74) Mandataire: **Cabinet Nony
11 rue Saint-Georges
75009 Paris (FR)**

(56) Documents cités:
KR-A- 20210 051 683    US-A1- 2020 333 246
US-A1- 2021 364 813

• JAEDUCK JANG ET AL: "Complex wavefront shaping for optimal depth-selective focusing in optical coherence tomography References and LinksHigh-speed scattering medium characterization with application to focusing light through turbid media", OPTICS EXPRESS, 30 January 2013 (2013-01-30), pages 2890 - 2902, XP055267151, Retrieved from the Internet <URL:https://opg.optica.org/oe/fulltext.cfm?uri=oe-21-3-2890&id=248842> [retrieved on 20160420], DOI: 10.1364/OE.21.002890
• KANG SUNGSAM ET AL: "High-resolution adaptive optical imaging within thick scattering media using closed-loop accumulation of single scattering", vol. 8, no. 1, 1 December 2017 (2017-12-01), XP055946530, Retrieved from the Internet <URL:https://www.nature.com/articles/s41467-017-02117-8.pdf> DOI: 10.1038/s41467-017-02117-8

**Description**

La présente invention concerne la focalisation d'un rayonnement lumineux dans une zone d'étude au sein d'un milieu diffusant.

Il est connu de mesurer la concentration de chromophores dans un tissu biologique, par exemple l'oxygénation des tissus cutanés, par spectroscopie par réflectance diffuse, aussi connue sous l'abréviation spectroscopie « DRS ».

La spectroscopie par réflectance diffuse *in vivo* met généralement en œuvre des sondes constituées de fibres optiques, couplées simultanément à une source d'émission lumineuse et à un spectromètre ou des photodiodes, pour transmettre un rayonnement lumineux dans une gamme définie de longueur d'onde (par exemple entre 400 nm et 1000 nm pour le visible et le proche infrarouge et entre 900 nm et 1700 nm pour l'infrarouge court) depuis la source d'émission lumineuse vers la surface d'un tissu, puis du tissu vers le spectromètre ou les photodiodes.

Au cours de leur trajet au sein du tissu, les photons du rayonnement lumineux sont absorbés et diffusés (*i.e.* déviés de leur trajectoire) par les constituants du tissu. Seule une partie des photons diffusés est réfléchie, c'est-à-dire qu'elle émerge par la surface du tissu par laquelle le rayonnement a été introduit dans le tissu, et peut ainsi être détectée.

La technique consistant à mesurer cette portion diffusée et réfléchie, aussi dénommée portion rétrodiffusée, est communément appelée technique de mesure en réflectance diffuse. Elle est distincte d'une technique de mesure en transmission dans laquelle la détection du rayonnement diffusé est effectuée du côté du tissu opposé à la source d'émission lumineuse. La spectroscopie en transmission ne permet d'accéder qu'à quelques régions spécifiques d'un corps, tel des doigts, orteils ou lobes d'oreille, du fait de l'absorption de la lumière dans l'épaisseur des tissus correspondants.

La spectroscopie DRS s'appuie sur le fait que les photons dans les longueurs d'onde du visible et du proche-infrarouge, soit entre 400 nm et 1700nm, ont la propriété d'interagir avec les constituants des tissus vivants. Ces interactions permettent donc l'extraction d'informations optiques relatives aux concentrations de ces constituants.

Dans le cas de la peau, les constituants qui diffusent les photons sont notamment les mélanosomes, le collagène et les cellules sanguines. Les constituants absorbant la lumière sont appelés chromophores. Les chromophores dans la gamme de longueur d'onde allant de 400 nm à 1700 nm sont entre autres l'eau, les lipides, la mélanine et l'hémoglobine (oxy et desoxygénée), et le glucose qui est généralement présent en faible concentration.

Plusieurs méthodes instrumentales sont connues pour séparer les phénomènes de diffusion et d'absorption de la lumière dans les tissus biologiques. On peut les distinguer par exemple par la nature de la modulation de la source d'émission lumineuse ou de la méthode de détection.

Une catégorie de systèmes connus met en œuvre une acquisition du rayonnement rétrodiffusé résolue en temps. De tels systèmes comportent une source d'émission d'un rayonnement laser d'une impulsion picoseconde ou femtoseconde avec un taux de répétition allant de 50 à 100 MHz. Les mesures se font à l'aide d'un système de comptage de photons corrélés dans le temps. Le rayonnement laser a été atténué et a subi un décalage temporel lors de son voyage dans le tissu biologique. Ce décalage temporel représente le "temps de vol" des photons durant leur interaction avec le tissu. Une telle technique résolue en temps est coûteuse et requiert une électronique associée complexe.

Une autre catégorie de systèmes connus met en œuvre l'émission d'une lumière continue en intensité, blanche ou monochromatique, et une détection du signal rétrodiffusé variant dans l'espace. L'intensité rétrodiffusée ou transmise par le tissu est mesurée grâce à un photomultiplicateur, une photodiode ou un spectromètre dans le cas d'un système fibré. Du fait du caractère diffusant des tissus biologiques, la lumière suit des chemins complexes entre la source d'émission lumineuse et la détection du signal rétrodiffusé. En outre, une portion de la lumière incidente peut être absorbée. Une unique mesure de l'atténuation de l'intensité lumineuse à une distance donnée de la source n'est donc pas suffisante pour distinguer les phénomènes de diffusion des phénomènes d'absorption. C'est pourquoi ces systèmes ont été améliorés en enregistrant la réflectance à plusieurs distances entre la source d'émission lumineuse et le récepteur sur le tissu étudié et analysent la variation de l'atténuation en fonction de la distance entre le point de mesure et la source d'émission lumineuse. De tels systèmes mettent ainsi en œuvre une méthode de mesure résolue spatialement. Il est aussi connu de mettre en œuvre une méthode dite de lumière structurée, qui se distingue de la méthode résolue spatialement décrite ci-dessus en ce que l'analyse de la réflectance est effectuée dans le domaine de Fourier. US 6,958,815 B2 et US 2013/0274612 A1 décrivent des exemples de mise en œuvre d'une méthode de lumière structurée.

Cependant, la mesure des chromophores par DRS est délicate du fait de l'hétérogénéité des tissus biologiques.

Les articles Petitdidier, N. A. (2018)., « Contact, high-resolution spatial diffuse reflectance imaging system for skin condition diagnosis », Journal of Biomedical Optics 23 (11), doi:10.1117/1.JBO.23.11.115003 et Koenig, A. N. (2021). « Contact, High-Resolution Spatial Diffuse Reflectance Imaging Systemfor Skin Condition Diagnosis: A First-in-Human Clinical trial », Journal of Biomedical Optics 26 (01), décrivent la mise en œuvre d'une matrice de détecteurs afin d'obtenir une courbe de décroissance de la réflectance mieux résolue. Ces auteurs accèdent ainsi à la composition des tissus biologiques dans un volume sondé par les photons collectés. Cependant, la caractérisation des tissus biologiques est alors limitée par la largeur du faisceau et le recouvrement de celui-ci avec la zone d'étude au sein du tissu. Par exemple, afin de mesurer une teneur en sucre, la zone d'étude est idéalement un des réseaux vasculaires du tissu cutané situés dans le derme réticulaire ou dans le derme papillaire, ou à défaut le liquide interstitiel. Ces réseaux vasculaires sont situés respectivement à environ 2 mm et 300 $\mu$m de profondeur de la surface de la peau, et présentent une section d'un diamètre inférieur à cent microns. Pour atteindre cette zone d'étude, le récepteur du rayonnement rétrodiffusé est distant d'environ 5 mm de la source d'émission lumineuse. L'enveloppe des trajectoires moyennes des photons diffusés depuis l'émetteur jusqu'au récepteur dans le tissu biologique présente alors une section supérieure au millimètre, supérieure au diamètre de la zone ciblée.

L'article Bykov et al. (2007). « Time gating in glucose sensing with ultrashort pulses. Advanced Laser Technologies. », Proc. SPIE 7022, Advanced Laser Technologies 2007, 70220X; doi :10.1117/12.804085 décrit une méthode en temps résolu pour pallier cette difficulté. Il décrit une fenêtre temporelle limitant le temps d'intégration des photons. Les photons ayant un temps de trajet trop long, respectivement trop court, correspondent à un trajet trop long, respectivement trop court, par rapport au trajet moyen des photons « balistiques » censés traverser le tissu pour aller directement dans la zone d'intérêt puis en ressortir. La méthode décrite dans Bykov et *al.* fonctionne correctement dans le visible où les détecteurs utilisés sont peu bruyants, mais pas dans l'infrarouge. Le bruit du détecteur provoque alors un déclenchement intempestif de celui-ci, temps pendant lequel le détecteur ne peut plus capter le rayonnement rétrodiffusé. Le filtrage temporel proposé n'est alors plus maîtrisé.

L'article Vellekoop et al. (2007), « Focusing coherent light through opaque stronglys cattering media », Optics Express, Vol.32, n°16, 2309-2311 décrit l'association d'un modulateur spatial de lumière, aussi connu sous l'abréviation « SLM », à un imageur placé de part et d'autre d'un tissu biologique. Le SLM produit une modulation du front d'onde afin de compenser la modulation du front d'onde engendrée par la traversée du tissu biologique. L'imageur placé à l'opposé du SLM par rapport au tissu biologique permet de rendre compte de la qualité, par exemple de focalisation, du rayonnement rétrodiffusé. Le signal produit par l'imageur est traité afin d'asservir le SLM. La méthode de Vellekoop présente des inconvénients. D'une part, elle est une méthode en transmission. D'autre part, il est impossible de garantir la qualité du rayonnement au sein du tissu biologique puisque cette dernière ne peut être constatée que sur l'imageur qui est placé en dehors du tissu.

WO 2020/201156 A1 décrit l'association d'un dispositif de mise en forme de front d'onde et une technique d'émission d'onde continue modulée en fréquence, dénommée sous l'abréviation onde « FMCW » pour des applications de spectrométrie.

L'article de Jaeduck Jang et al. : « Complex wavefront shapingfor optimal depth-selective focusing in optical coherence tomography », Optics Express, vol. 21, no. 3, 30 janvier 2013, p. 2890-2902, décrit une méthode pour exploiter la diffusion multiple de la lumière en façonnant le front d'onde incident pour la tomographie par cohérence optique.

KR 2021 0051683 A décrit un dispositif d'imagerie du type à balayage focalisé pour imager un objet cible dans un échantillon qui induit une aberration.

Il existe donc un besoin pour focaliser précisément un rayonnement lumineux sur une zone d'étude dans un milieu diffusant, préalablement à une mesure dans la zone d'étude au moyen d'un rayonnement lumineux rétrodiffusé par le milieu diffusant, et en s'affranchissant des inconvénients de l'art antérieur cités ci-dessus.

L'invention propose un dispositif pour focaliser un rayonnement lumineux dans une zone d'étude au sein d'un milieu diffusant, le dispositif comportant :

- une source d'émission lumineuse configurée pour émettre un rayonnement lumineux cohérent présentant au moins une longueur d'onde comprise entre 400 nm et 1700 nm, le rayonnement lumineux étant balayé en fréquence,
- un séparateur de faisceau configuré pour séparer le rayonnement lumineux émis par la source d'émission lumineuse en un rayonnement de sonde et en un oscillateur local respectivement,
- une unité de modulation optique pour modifier l'angle d'incidence sur un milieu diffusant d'au moins un des faisceaux du rayonnement de sonde,
- une unité de détection optique comportant un récepteur d'onde pour collecter l'oscillateur local et au moins une partie du rayonnement de sonde diffusé et réfléchi par le milieu diffusant, dite rayonnement de sonde collecté, et un générateur d'onde configuré pour générer un battement électromagnétique par mélange du rayonnement de sonde collecté et de l'oscillateur local, la source d'émission lumineuse et l'unité de détection optique étant disposés d'un même coté du milieu diffusant, et
- une unité de commande configurée pour asservir l'unité de modulation optique afin de maximiser la valeur $q(f_{moy})$ de la densité d'énergie spectrale de puissance du battement électromagnétique $q(f)$ à la fréquence moyenne du battement électromagnétique $f_{moy}$.

En comparant le temps de trajet de l'oscillateur local et celui du rayonnement de sonde rétrodiffusé par le milieu optique entre la source d'émission lumineuse et l'unité de détection optique puis en modulant de façon appropriée le rayonnement de sonde en fonction de cette comparaison, l'invention permet par mise en œuvre d'une mesure en réflexion, de focaliser le rayonnement de sonde spécifiquement dans la zone d'étude, notamment en réduisant la taille du rayonnement de sonde au plus près de la taille de la zone d'étude. En outre, la mise en œuvre du dispositif peut être mise en œuvre aussi bien dans le visible que dans l'infrarouge.

La fréquence moyenne $f_{moy}$ du battement électromagnétique est caractéristique de la durée moyenne $T_{moy}$ du trajet des photons du rayonnement de sonde pour traverser le milieu diffusant entre la source d'émission lumineuse et le récepteur d'onde. Notamment, elle est proportionnelle à la durée du trajet moyen $T_{moy}$ du rayonnement de sonde dans le milieu diffusant. Elle est déterminée à partir de la densité spectrale de puissance du battement électromagnétique et est égale à la moyenne arithmétique des fréquences $f_i$ du spectre fréquentiel du battement électromagnétique, pondérée par

les valeurs $q_i$ de la densité spectrale pour chacune des puissances $f_i$ du spectre, *i.e.* $f_{moy} = \frac{\sum f_i q_i}{\sum q_i}$.

De préférence, le dispositif comporte une unité d'analyse pour déterminer la largeur à mi-hauteur de la densité spectrale de puissance du battement électromagnétique. L'unité de commande est de préférence configurée pour asservir l'unité de modulation optique afin de minimiser la largeur à mi-hauteur $\Delta f$ de la densité spectrale de puissance du battement électromagnétique. Avantageusement, la minimisation de la largeur à mi-hauteur de la densité spectrale de puissance induit une concentration de l'énergie du battement électromagnétique autour de la fréquence moyenne dudit battement. Elle est ainsi une option pour maximiser la valeur $q(f_{moy})$ de la densité d'énergie spectrale de puissance du battement électromagnétique $q(f)$ à la fréquence moyenne du battement électromagnétique $f_{moy}$. En particulier, elle peut être bien adaptée lorsque la densité spectrale de puissance du battement électromagnétique présente une forme de courbe en cloche, notamment est une courbe gaussienne. De préférence, le rayonnement lumineux est un laser, de préférence accordable. Le rayonnement lumineux est balayé, de préférence linéairement, en fréquence.

De préférence, l'unité de modulation optique est un modulateur spatial de lumière ou un réseau optique à commande de phase. De préférence, l'unité de modulation optique est un modulateur spatial de lumière. Le modulateur spatial de lumière peut être un modulateur spatial de lumière en phase, ou un modulateur spatial de lumière en amplitude ou un modulateur spatial de lumière en réflexion.

Le modulateur spatial de lumière peut comporter une matrice de pixels comportant chacun des cristaux liquides, et un organe d'alimentation électrique configuré pour appliquer une tension électrique déterminée indépendamment à chaque pixel. Ainsi, chaque pixel peut modifier indépendamment la polarisation de la portion du rayonnement de sonde qui le traverse, indépendamment des pixels qui en sont adjacents, et notamment dévier ladite portion du rayonnement de sonde.

De préférence, le séparateur de faisceau est configuré pour que la phase et la fréquence fondamentale de l'oscillateur local et la phase et la fréquence fondamentale du rayonnement de sonde soient égales respectivement.

De préférence, le séparateur de faisceau est configuré pour que l'amplitude de l'oscillateur local soit plus de deux fois, voire plus de cinq fois, voire plus de dix fois, supérieure à l'amplitude du rayonnement de sonde. De cette façon, l'unité d'analyse peut s'affranchir de la contribution à l'intensité totale du battement électromagnétique provenant de la multiplication de l'amplitude de l'onde secondaire avec l'amplitude de chacun des faisceaux du rayonnement de sonde collecté.

Le séparateur de faisceau peut être un cube séparateur de faisceau ou une lame séparatrice de faisceau, ou un multiplexeur.

Le dispositif peut comporter un guide d'onde pour conduire l'oscillateur local du séparateur de faisceau jusqu'à l'unité de détection optique.

De préférence, le guide d'onde est une fibre optique.

Le guide d'onde peut être configuré pour que la longueur optique du trajet de l'oscillateur local entre le séparateur de faisceau et l'unité de détection optique soit égale à la longueur optique du trajet moyen du rayonnement de sonde dans le milieu diffusant entre le séparateur de faisceau et l'unité de détection optique. La longueur optique dudit trajet moyen peut aisément être calculée à partir de simulations Monte Carlo. Une interaction constructive entre l'oscillateur local et les faisceaux du rayonnement de sonde collectée est ainsi obtenue. Avantageusement, suite à la focalisation au moyen du dispositif selon l'invention, une légère modification du milieu diffusant résulte en une variation du trajet optique moyen suivi par la lumière dans le milieu diffusant. Cette modification du milieu diffusant peut alors être aisément constatée par des changements d'interférences. Par exemple, une composition formée d'eau et de 2 % en masse d'Intralipide® peut être considérée comme présentant sensiblement les mêmes propriétés d'absorption et de diffusion qu'un tissu biologique, et l'homme du métier sait aisément déterminer la longueur du trajet moyen des photons entre la source d'émission lumineuse et le récepteur d'ondes dans un tel milieu. Il peut alors aisément adapter le guide d'onde en conséquence.

Selon une variante, le guide d'onde peut être configuré pour que la longueur optique du trajet de l'oscillateur local entre le séparateur de faisceau et le système de détection optique soit supérieure à la longueur du trajet moyen du rayonnement de sonde à travers le milieu diffusant entre le séparateur de faisceau et l'unité de détection, de façon à maximiser le rapport entre l'intensité du battement électromagnétique sur le bruit du récepteur d'onde.

En particulier, selon cette variante, le récepteur d'onde peut comporter une photodiode à jonction PIN, notamment formée de germanium, qui présente notamment un bruit de grenaille pour une fréquence coin $f_c$, par exemple comprise entre 10 kHz et 100 kHz, en particulier d'environ 10 kHz. La longueur du trajet de l'oscillateur local entre le séparateur de faisceau et le système de détection optique peut être telle que la fréquence du battement électromagnétique soit supérieure à la fréquence coin, par exemple égale à deux fois la fréquence coin.

Le dispositif peut comporter un support et l'unité de détection optique, en particulier le détecteur d'onde, et la source d'émission lumineuse peuvent être portées par le support. En particulier, elles peuvent être portées par une même face du support, de préférence opposée à la face du support en regard du milieu diffusant.

Notamment, l'unité de modulation optique peut s'étendre entre la source d'émission lumineuse et le support.

Le support est de préférence en un matériau transparent au rayonnement lumineux émis par la source d'émission lumineuse.

Par ailleurs, le matériau constitutif du guide d'onde peut présenter un indice optique variant avec la température et adapté à la variation de l'indice optique du milieu diffusant avec la température. La plage de températures sur laquelle l'indice optique peut présenter une telle variation peut être choisie en fonction de l'application à laquelle le dispositif est destiné. Ainsi, la différence entre la longueur du trajet optique de l'oscillateur local dans le guide d'onde et la longueur du trajet optique moyen du rayonnement de sonde dans le milieu reste sensiblement constante avec une variation de température. En particulier, notamment sur une plage de température comprise entre 25 °C et 38 °C correspondant par exemple à la température d'étude d'un tissu biologique, le rapport

[Math 1]

$$\left| \frac{n_{OL}(T)L_{OL} - n_{MD}(T)L_{MD}}{n_{OL}(T)L_{OL}} \right|$$

peut être inférieur à 0,30, voire inférieur à 0,15, mieux inférieur à 0,05.

$n_{OL}(T)$ est l'indice optique du matériau constitutif du guide d'onde, $L_{OL}$ est la longueur du trajet de l'oscillateur local, $n_{MD}(T)$ est l'indice optique du matériau du milieu diffusant et $L_{MD}$ est la longueur du trajet optique moyen du rayonnement de sonde dans le milieu diffusant.

En particulier, le milieu diffusant peut comporter de l'eau, par exemple pour plus de 50 % de sa masse, et notamment être un tissu biologique, et le matériau constitutif du guide d'onde présente un indice optique dont la variation avec la température est comprise entre -100*10$^{-6}$ °C$^{-1}$ et -200*10$^{-6}$ °C$^{-1}$, sur une plage de température comprise entre 25 °C et 38 °C. De préférence, le matériau constitutif du guide d'onde étant est parmi le silicium, le nitrure de silicium, l'oxyde de silicium SiO$_2$ et leurs mélanges. De préférence, le matériau constitutif du guide d'onde est le silicium qui présente une variation $^{dn}/_{dT}$ de l'indice optique n avec la température T qui est égal à -160*10$^{-6}$°C$^{-1}$ sur une plage de température comprise entre 10 °C et 50 °C. Une telle variation est proche de celle de l'eau qui est égale à 110*10$^{-6}$°C$^{-1}$.

Le dispositif peut comporter une sonde thermique pour mesurer la température du milieu diffusant, et une unité de régulation thermique configurée pour modifier l'indice optique du guide optique en fonction de la température mesurée par la sonde thermique, notamment de telle sorte que l'indice optique du guide d'onde soit égal à l'indice optique du milieu diffusant. En particulier, l'unité de régulation thermique peut être configurée pour appliquer une tension électrique, dépendante de la température mesurée par la sonde de température, sur le guide optique afin de modifier l'indice optique dudit guide optique, comme cela est par exemple décrit dans l'article « Strong electro-optical modulation enhancement in a slow wave corrugated waveguide », A. Brimont et al., Optics Express, Vol. 17, Issue 11, pages 9204-9211 (2009), doi:10.1364/OE.17.009204 peut être mise en œuvre. La sonde thermique peut être au contact du milieu diffusant.

Par ailleurs, le dispositif peut comporter plusieurs récepteurs d'onde et être configuré pour que chaque récepteur d'onde reçoive séquentiellement l'oscillateur local. En particulier, il peut comporter plusieurs guides d'onde pour amener chacun séquentiellement l'oscillateur local du séparateur de faisceau à l'un des récepteurs d'ondes correspondant. De préférence, les longueurs des guides d'ondes sont différentes, afin de générer des battements électromagnétiques présentant des interférences différentes.

Le générateur d'onde comporte de préférence un mélangeur d'ondes et un soustracteur comportant deux photodiodes montées électriquement en opposition pour extraire la partie modulée du battement électromagnétique. Avantageusement, un tel générateur d'ondes permet de réduire le bruit résultant du mélange du rayonnement de sonde collecté et de l'oscillateur local.

Par ailleurs, la détermination de la largeur à mi-hauteur de la densité spectrale de puissance du battement électromagnétique est de préférence obtenue par un traitement comportant une transformée de Fourier du battement électromagnétique.

L'invention concerne encore un appareil comportant le dispositif selon l'invention, l'appareil étant choisi parmi un appareil de télédétection, notamment de type LIDAR, et un appareil de spectroscopie par réflectance diffuse.

Par ailleurs, l'invention concerne un procédé de focalisation d'un rayonnement lumineux dans une zone d'étude au sein d'un milieu diffusant au moyen du dispositif selon l'invention, le procédé comportant :

- l'émission d'un rayonnement lumineux cohérent, le rayonnement lumineux étant balayé en fréquence,
- la séparation du rayonnement lumineux en un oscillateur local et un rayonnement de sonde,
- l'irradiation au moyen du rayonnement de sonde d'une partie du milieu diffusant contenant la zone d'étude,
- la détection d'au moins une partie du rayonnement de sonde diffusé et réfléchi par le milieu diffusant, dite rayonnement de sonde collecté, et de l'oscillateur local,
  l'émission du rayonnement lumineux et la détection du rayonnement de sonde collecté étant effectuées d'un même côté du milieu diffusant,
- le mélange du rayonnement de sonde collecté et de l'oscillateur local pour former un battement électromagnétique, et
- la modulation optique du rayonnement de sonde par modification de l'angle d'incidence sur le milieu diffusant d'un ou plusieurs faisceaux du rayonnement de sonde, afin de maximiser la valeur $q(f_{moy})$ de la densité d'énergie spectrale de puissance du battement électromagnétique $q(f)$ à la fréquence moyenne du battement électromagnétique $f_{moy}$.

De préférence, le procédé comporte la détermination de la largeur à mi-hauteur $\Delta f$ de la densité spectrale de puissance du battement électromagnétique. De préférence, la modulation optique du rayonnement de sonde est opérée de façon à minimiser la largeur à mi-hauteur de la densité spectrale de puissance du battement électromagnétique.

Le milieu diffusant peut être solide ou fluide, notamment liquide et/ou gazeux. Il peut comporter des éléments solides dispersés dans un fluide peut comporter des gouttelettes d'un liquide en suspension dans un gaz.

Le milieu diffusant peut être un tissu biologique. Le tissu biologique peut notamment être une partie d'un corps animal, en particulier d'un corps humain, par exemple une peau. En variante, le tissu biologique peut être végétal. Il est bien entendu que le procédé de focalisation du rayonnement n'a aucune visée thérapeutique.

Le milieu diffusant peut être une suspension de gouttelettes d'un liquide, notamment d'eau, dans un gaz, notamment l'air. Le milieu diffusant peut être un brouillard ou une brume.

Lorsque le milieu diffusant est solide, le procédé peut comporter la mise en contact du support avec le milieu diffusant.

L'invention concerne encore une méthode de spectroscopie par réflectance diffuse, la méthode comportant :

- la mise en œuvre du procédé de focalisation selon l'invention, le milieu diffusant étant un tissu biologique et la zone d'étude comportant des chromophores,
- l'émission d'un rayonnement de sonde spectroscopique vers l'unité de modulation optique afin de focaliser le rayonnement de sonde spectroscopique sur la zone d'intérêt, et
- la détection de la partie du rayonnement réémis par les chromophores sensibles au rayonnement de sonde spectroscopique, qui est diffusée et réfléchie par le milieu diffusant.

La méthode de spectroscopie n'est pas une méthode thérapeutique. Elle vise seulement à détecter la présence de constituants spécifiques du tissu, et optionnellement en mesurer leur teneur.

Les chromophores peuvent être choisis parmi ceux constituant le tissu à examiner. Par exemple le tissu est une peau et les chromophores peuvent être choisis parmi l'hémoglobine, un sucre, en particulier le glucose, l'eau, un lipide, la mélanine, la bilirubine et leurs mélanges.

En variante, elle est par exemple mise en œuvre pour mesurer la glycémie dans le sang s'écoulant dans un réseau vasculaire sous-cutané.

L'invention concerne enfin une méthode de télédétection d'un objet dans une suspension, dans un gaz, de gouttelettes d'un liquide, la méthode comportant :

- la mise en œuvre du procédé de focalisation selon l'invention, le milieu diffusant étant une suspension dans un gaz de gouttelettes d'un liquide et la zone d'étude contenant l'objet,
- l'émission d'un rayonnement de sonde de télédétection vers l'unité de modulation optique afin de focaliser le rayonnement de sonde de télédétection dans la zone d'intérêt, et
- la détection de la partie du rayonnement réémis par l'objet qui est diffusée et réfléchie par le milieu diffusant.

Avantageusement, la méthode de télédétection permet d'améliorer la détection d'un objet dans un milieu diffusant, en tenant compte de l'effet diffusant dudit milieu sur le trajet des photons émis par la source d'émission lumineuse et atteignant le récepteur d'onde.

L'invention pourra être mieux comprise à la lecture de la description détaillée qui va suivre et à l'examen du dessin annexé, dans lequel

[fig. 1] représente de manière schématique un exemple de mise en œuvre de spectroscopie par réflectance diffuse connu de l'art antérieur,
[fig. 2] représente un exemple de dispositif selon l'invention,

[fig. 3] est une courbe représentant le balayage en fréquence du rayonnement lumineux émis par le dispositif de la figure 2,

[fig 4] est une courbe représentant schématiquement la distribution spectrale de puissance du battement électromagnétique mesuré par le dispositif de la figure 2,

[fig 5] illustre de manière schématique une technique d'asservissement d'un modulateur spatial de lumière,

[fig 6] représente schématiquement un générateur d'onde d'un exemple de dispositif selon l'invention,

[fig 7] représente un autre exemple de dispositif selon l'invention,

[fig 8] représente schématiquement autre exemple de dispositif selon l'invention, et

[fig 9] illustre schématiquement une utilisation d'un exemple de dispositif selon l'invention pour la télédétection d'un objet.

Dans les figures, les différents éléments constituant le dispositif selon l'invention ainsi que le milieu diffusant ne sont pas représentés à l'échelle, par souci de clarté du dessin.

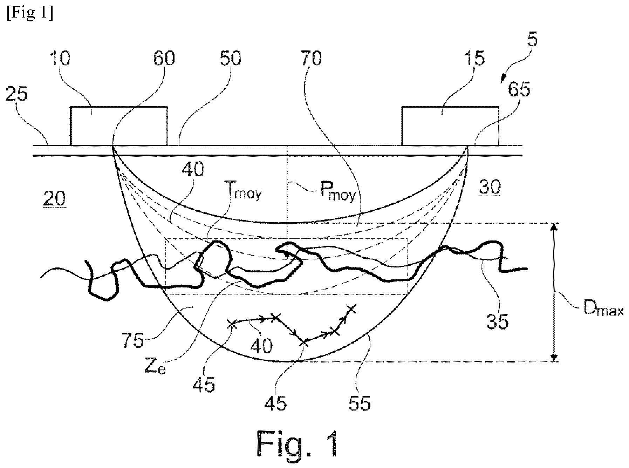

On a représenté sur la figure 1 de manière schématique un exemple d'appareil 5 de spectroscopie par réflectance diffuse connu de l'art antérieur.

L'appareil comporte une source d'émission 10 d'un rayonnement laser et un récepteur d'onde 15 qui sont disposés tous les deux d'un même côté d'un tissu biologique 20, qui est par exemple un morceau d'un corps comportant un épiderme 25, un derme et un réseau vasculaire sous cutané 35.

La source d'émission 10 est agencée de telle sorte que le rayonnement laser 40 est dirigé vers le tissu biologique. En pénétrant dans le tissu biologique, le rayonnement laser est diffusé par de multiples sources de diffusion 45 qui sont par exemple les mélanosomes, le collagène et les cellules sanguines. Une partie du rayonnement diffusé est ainsi réfléchi sous l'effet des déviations multiples vers la surface 50 par laquelle il a pénétré. Cette partie du rayonnement incident est dénommée rayonnement rétrodiffusé.

L'ensemble des faisceaux du rayonnement laser rétrodiffusé qui est collecté par le récepteur est contenu dans une enveloppe 55. L'enveloppe 55 comporte l'ensemble statistique des trajectoires des photons collectés par le récepteur d'onde 15. Elle présente une forme qui s'élargit à partir du point de contact 60 du rayonnement laser incident avec la surface du tissu biologique jusqu'à atteindre un diamètre maximal $D_{max}$ avant de s'effiler progressivement jusqu'au point de détection 65.

L'homme du métier sait estimer, en fonction des propriétés d'absorption et de diffusion du tissu biologique, le trajet moyen $T_{moy}$ des photons entre la source d'émission lumineuse et le détecteur et la profondeur moyenne $P_{moy}$ que le rayonnement atteint dans le tissu biologique.

De cette façon, il sait comment sonder une zone d'étude $Ze$, délimitée de manière schématique par un rectangle en pointillés sur la figure 1, au sein du tissu biologique, par exemple qui contient le réseau vasculaire. De cette façon, il peut mesurer la quantité de photons d'énergie spécifique réémis par exemple par le glucose, de façon à estimer la glycémie.

Comme cela apparaît sur la figure 1, une partie 70 du rayonnement rétrodiffusé qui atteint le récepteur d'onde, et qui est contenue dans l'enveloppe 55, n'atteint pas la zone d'étude. Une autre partie 75 a traversé la zone d'étude mais est formée de photons dont la majorité du trajet a été effectuée hors de la zone d'étude.

Ces parties du rayonnement captées par le récepteur génèrent un bruit dans la mesure de spectroscopie en réflectance diffuse qu'il convient de minimiser. Pour cela, l'invention vise à réduire le diamètre de l'enveloppe 55 pour maximiser la portion interagissant dans la zone d'étude.

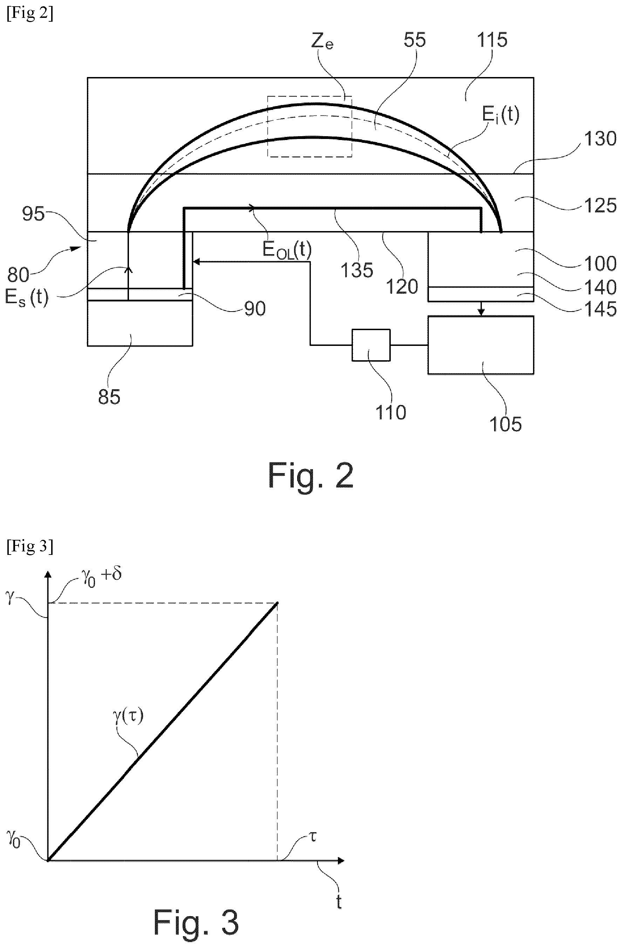

La figure 2 illustre un exemple de dispositif 80 selon l'invention pour focaliser un rayonnement dans une zone d'étude $Z_e$ telle que celle présentée sur la figure 1.

Le dispositif 80 comporte une source d'émission lumineuse 85, un séparateur de faisceau 90, une unité de modulation optique 95, une unité de détection optique 100, une unité d'analyse 105 et une unité de commande 110.

La source d'émission lumineuse 85 et l'unité de détection optique 100 sont disposées d'un même côté d'un milieu diffusant 115, qui dans l'exemple illustré est le même milieu biologique que celui illustré sur la figure 1. Par exemple, elles sont portées par une face 120 d'un support 125 dont une face opposée 130 est au contact du tissu biologique.

Par ailleurs, le dispositif comporte un guide d'onde 135 disposé entre le séparateur de faisceau et l'unité de détection optique. Le guide d'onde est de préférence une fibre optique, qui peut être logée dans un conduit ménagé dans le support.

Afin focaliser le rayonnement sur la zone d'étude $Z_e$, la source d'émission 85 émet un rayonnement lumineux $E(t)$ en direction du milieu diffusant 115, de préférence un rayonnement laser, d'équation

[math 2]

$$E(t) = \|E\|e^{i2\pi vt}$$

où $\|E\|$ et $v$ sont respectivement l'intensité et la fréquence du rayonnement.

Le rayonnement lumineux est balayé linéairement en fréquence, c'est-à-dire que sa fréquence ν évolue en fonction du temps t de manière périodique entre $\nu_0$ et $\nu_0 + \delta$ comme cela est représenté sur la figure 3 selon l'équation

[math 3]

$$\nu = \nu_0 + \frac{\delta t}{\tau}$$

Le rayonnement lumineux est séparé par le séparateur de faisceau 90 en un rayonnement de sonde $E_s(t) = \|E_s\|e^{i2\pi\nu t}$, qui est dirigé vers le milieu diffusant 115, et en un oscillateur local $E_{OL}(t) = \|E_{OL}\|e^{i2\pi\nu t}$ qui est guidé par le guide d'onde 135 jusqu'à l'unité de détection optique 100. Le rayonnement de sonde et l'oscillateur local sont tels que $\|E\| = \|E_s\| + \|E_{OL}\|$, avec de préférence $\|E_{OL}\| > \|E_s\|$.

Avant de pénétrer dans le milieu diffusant 115, le rayonnement de sonde $E_s(t)$ traverse l'unité de modulation optique 95.

Dans une première étape, l'unité de modulation optique 80 ne modifie pas le rayonnement de sonde $E_s(t)$.

Le rayonnement de sonde $E_s(t)$ pénètre ensuite dans le milieu diffusant 115. Les faisceaux qui le constituent sont alors individuellement déviés par les sources diffusantes du milieu diffusant.

L'unité de détection optique 100 comporte un récepteur d'ondes 140 pour collecter une partie du rayonnement de sonde ainsi diffusée et réfléchie par le milieu diffusant. Cette partie est contenue dans une enveloppe 55 telle que décrite pour illustrer la figure 1.

Chacun des faisceaux $E_i(t)$ contenu dans l'enveloppe est collecté par le récepteur d'ondes et est déphasé par rapport à l'oscillateur d'un déphasage $\varphi_i$ qui caractérise la durée supplémentaire induite par le milieu diffusant 115 sur le faisceau $E_i(t)$ du rayonnement de sonde pour atteindre le récepteur d'ondes 100 par rapport à l'oscillateur local $E_{OL}(t)$.

Par ailleurs, l'unité de détection optique comporte un générateur d'onde 145 pour mélanger les faisceaux $E_i(t)$ avec l'oscillateur local afin de générer un battement électromagnétique.

L'intensité $I$ du battement électromagnétique est exprimée par l'équation

[math 4]

$$
\begin{aligned}
I &= \left(E_{OL} + \sum_i E_i\right)\left(E_{OL} + \sum_i E_i\right)^* \\
&= E_{OL}E_{OL}^* + 2\sum_i Re(E_i E_{OL}^*) \\
&= E_{OL}E_{OL}^* + 2\|E_i E_{OL}^*\|cos(\varphi_i)
\end{aligned}
$$

En considérant que $T_{OL}$ est la durée du trajet de l'oscillateur local $E_{OL}(t)$ pour atteindre le récepteur d'onde, et que $T_i$ est la durée du trajet du faisceau $E_i(t)$ pour atteindre le récepteur d'onde 140 en traversant le milieu diffusant 115, l'oscillateur local $E_{OL}(t)$ et chaque faisceau $E_i(t)$ sont à l'entrée du récepteur d'onde tels que

[math 5]

$$
\begin{aligned}
E_{OL}(t) &= \|E_{OL}\|e^{i2\pi\nu(t-T_{OL}).t} \\
E_i(t) &= \|E_i\|e^{i2\pi\nu(t-T_i).t}
\end{aligned}
$$

Ainsi, l'intensité $I$ du battement électromagnétique est exprimée par l'équation

[math 6]

$$I = E_{OL}E_{OL}^* + 2\sum_i \|E_i\| \|E_{OL}\|cos(2\pi f_i.t + \varphi_i)$$

Avec la fréquence $f_i$ du faisceau i étant:

[math 7]

$$f_i = \delta . \frac{T_{ol} - T_i}{\tau}$$

La fréquence moyenne $f_{moy}$ du battement électromagnétique est donc proportionnelle à la durée du trajet moyen $T_{moy}$ du rayonnement de sonde dans le tissu biologique.

Une analyse en fréquence du battement électromagnétique est ensuite réalisée par l'unité d'analyse 105 afin de déterminer la densité spectrale de puissance du battement électromagnétique.

La densité spectrale de puissance du battement électromagnétique est le carré du module de la transformée de Fourier du battement électromagnétique, divisé par la largeur de la bande spectrale dudit battement. Elle représente la répartition fréquentielle de la puissance du battement électromagnétique suivant les fréquences qui le composent.

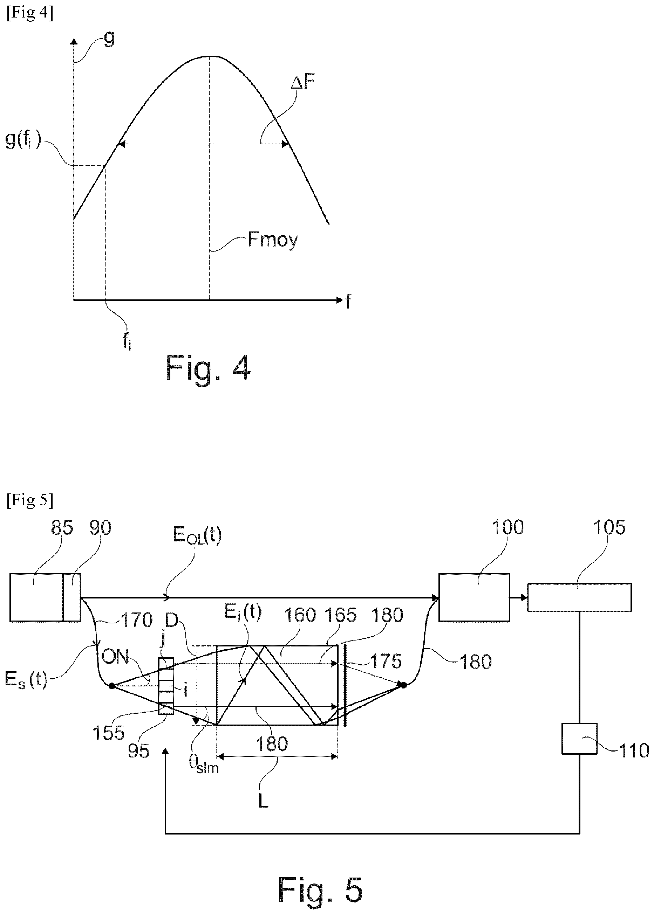

La figure 4 fournit un exemple schématique de courbe de densité spectrale d'un battement électromagnétique, dans laquelle la puissance $q_i$ associée à la composante de fréquence $f$ est représentée en fonction de la fréquence $f_i$. L'unité d'analyse détermine la fréquence moyenne $f_{moy}$ ainsi que la largeur à mi-hauteur $\Delta f$ du battement électromagnétique.

La fréquence moyenne $f_{moy}$ du battement électromagnétique est caractéristique de la durée moyenne $T_{moy}$ du trajet des photons pour traverser le milieu biologique entre la source d'émission lumineuse et le récepteur d'onde et la largeur à mi-hauteur $\Delta f$ est liée au diamètre maximal $D_{max}$ de l'enveloppe. Ainsi, plus diamètre maximal $D_{max}$ de l'enveloppe est élevé, plus la largeur à mi-hauteur $\Delta f$ l'est aussi.

La largeur à mi-hauteur $\Delta f$ est transmise à l'unité de commande en vue d'asservir l'unité de modulation optique.

Dans une deuxième étape, sur la base de largeur à mi-hauteur $\Delta f$ déterminée par l'unité d'analyse 105, l'unité de modulation optique est réglée de manière à ce que lors de son passage dans l'unité de modulation optique, au moins une partie du rayonnement de sonde soit déviée, de telle sorte que le diamètre maximal $D_{max}$ de l'enveloppe formée par les faisceaux du rayonnement de sonde diffusés et réfléchis vers le récepteur d'onde soit minimisé.

Ainsi, lors de cette deuxième étape, après avoir traversé le milieu diffusant, la largeur à mi-hauteur $\Delta f$ de la densité spectrale de puissance du rayonnement de sonde $\Delta f$ est ainsi réduite. Le réglage de l'unité de modulation optique peut être poursuivi en reproduisant un ou plusieurs cycles de réduction de la largeur à mi-hauteur $\Delta f$ de la densité spectrale de puissance du rayonnement de sonde.

L'unité de modulation optique tel qu'illustré sur la figure 2 peut notamment être un modulateur spatial de lumière.

La figure 5 illustre de manière schématique une technique d'asservissement d'un tel modulateur spatial de lumière 155. Le dispositif de la figure 5 diffère de celui illustré sur la figure 2 en ce que le milieu représenté est un barreau 160 de verre d'indice n, d'une longueur L, d'un diamètre D et qui présente un rapport de la longueur sur le diamètre L/D supérieur à 10. Dans l'exemple illustré sur cette figure 5, le milieu est non diffusant. Toutefois, du fait de son rapport L/D élevé, de nombreuses réflexions de Fresnel du rayonnement de sonde ont lieu sur les parois 165 du barreau, de la même façon qu'un milieu diffusant engendre aussi de nombreuses réflexions.

Le rayonnement laser émis par la source d'émission lumineuse est séparé par un séparateur 90 en un oscillateur local $E_{OL}(t)$, qui est dirigé directement vers l'unité de détection d'onde, et en un rayonnement de sonde $E_s(t)$. Le rayonnement laser présente une variation temporelle de la fréquence du type $\nu = \nu_0 + \dfrac{\delta t}{\tau}$ telle que décrite précédemment.

Dans une première phase, le rayonnement de sonde $E_s(t)$ est dirigé vers le barreau de verre à travers une fibre optique d'amenée 170 présentant une ouverture numérique ON puis en traversant et sans être polarisé par le modulateur spatial de lumière.

Le rayonnement de sonde qui pénètre dans le barreau comporte des faisceaux $E_i(t)$ qui sont réfléchis par les parois 165 du barreau 160. En sortie du barreau, ces faisceaux réfléchis sont collectés au moyen d'un système optique 175 puis focalisés vers une fibre optique de sortie 180 jusqu'à l'unité de détection optique 100. Un battement électromagnétique est généré par mélange de l'oscillateur local et du rayonnement de sonde collecté, et la largeur à mi-hauteur $\Delta f$ est déterminée par l'unité d'analyse.

La longueur $l$ du trajet parcouru dans le barreau par un faisceau du rayonnement de sonde qui est passé par le pixel i du modulateur spatial de lumière 155 et qui est réfléchi par les parois 165 du barreau 160 peut être approximée comme étant égale à :

[math 8]

$$l = \frac{L}{\cos \theta_i}$$

et $\theta_i$ l'angle d'incidence du rayon associé au pixel i sur la paroi du barreau. Cet angle $\theta_i$ est tel que

[math 9]

$$\theta_i = \frac{1}{n} \left( \theta_i^{slm} + \theta_i^f \right)$$

avec $\theta_i^{slm}$ étant l'angle de déflexion produite par le pixel i sur un faisceau du rayonnement de sonde et $\theta_i^f$ étant l'angle d'incidence du rayonnement en sortie de la fibre optique et qui atteint le pixel *i*, et qui est inférieure à l'ouverture numérique de la fibre optique ( $\left| \theta_i^f \right| <$ ON).

La fréquence dans le battement électromagnétique résultant est alors égale à :

[math 10]

$$f_i = \delta . \frac{T_{ol} - T_i}{\tau} = \frac{\delta}{\tau} . (T_{ol} - \frac{nL}{c.cos\theta_i})$$

avec c étant la vitesse de la lumière dans le vide.

Ainsi, lorsque le modulateur spatial de lumière n'induit aucune déviation des rayons lumineux, la fréquence $f_i$ décroit avec une augmentation de l'angle de sortie de fibre optique $\theta_i^f$ .

Dans l'exemple illustré sur la figure 5, la largeur à mi-hauteur Δ*f* peut être estimée par l'équation suivante :

[math 11]

$$\Delta f = \frac{\delta}{\tau} . \frac{nL}{c} . \left[ \frac{1}{\cos\left(\frac{1}{n} . (\theta_j^{slm} + ON)\right)} - \frac{1}{\cos\left(\frac{1}{n} . \theta_i^{slm}\right)} \right]$$

Dans cette expression :

- le pixel i est disposé au centre de la matrice de pixels du modulateur spatial de fréquence et est coaxial avec l'axe de la fibre optique d'amenée. Pour ce pixel i, l'angle d'incidence $\theta_i^f$ est donc nulle.
- le pixel *j* est un pixel disposé à distance du centre et pour lequel l'angle d'incidence du faisceau en sortie de la fibre optique d'amenée est égal à l'ouverture numérique ( $\theta_i^f = -ON$ )

Durant la première phase, $\theta_j^{slm}$ et $\theta_i^{slm}$ sont nuls, le modulateur spatial de lumière ne dévient pas le rayonnement de sonde, si bien que la largeur à mi-hauteur Δ*f* peut être approximée par :

[math 12]

$$\Delta f = \frac{\delta}{\tau} . \frac{nL}{c} . \frac{1}{\cos\left(\frac{1}{n} . ON\right)}$$

Durant la deuxième phase, dans l'exemple schématique illustré, la largeur à mi-hauteur peut être minimisée, en appliquant une déviation angulaire du faisceau interagissant avec le pixel *j* opposée à l'ouverture numérique, *i.e.* en choisissant

[math 13]

$$\begin{cases} \theta_j^{slm} = -ON \\ \theta_i^{slm} = 0 \end{cases}$$

Une telle minimisation peut être opérée par une technique de minimisation bien connue de l'homme du métier, par exemple par une technique de moindre carré.

De cette façon, après asservissement du modulateur spatial de lumière, le trajet de chaque faisceau du rayonnement de sonde dévié par le modulateur spatial de lumière est sensiblement parallèle au trajet moyen des photons, comme illustré par la référence 180.

Dans l'exemple illustré, l'unité de modulation optique est un modulateur spatial de lumière, mais un réseau optique à commande de phase pourrait être mis en œuvre en lieu et place du modulateur spatial de lumière.

L'exemple schématique ci-dessus met ainsi en évidence une technique pour asservir un modulateur spatial de lumière en fonction de la détermination de la densité spectrale de puissance du battement électromagnétique. L'homme du métier sait aisément mettre en œuvre une technique similaire pour asservir une unité de modulation optique et minimiser la largeur à mi-hauteur de la densité spectrale de puissance d'un battement électromagnétique résultant de l'interaction d'un faisceau sonde avec un milieu non diffusant, bien que cette largeur à mi-hauteur soit difficile voire impossible à exprimer de manière analytique.

Ainsi, dans l'exemple de réalisation présenté sur la figure 2, dans une deuxième étape, l'unité d'analyse transmet la valeur de la largeur à mi-hauteur à l'unité de commande qui peut ainsi appliquer une tension spécifique à chacun des pixels du modulateur spatial de lumière pour dévier un faisceau avec lequel le pixel interagit de manière à réduire le diamètre maximal de l'enveloppe, et ainsi focaliser le rayonnement de sonde dans la zone d'étude.

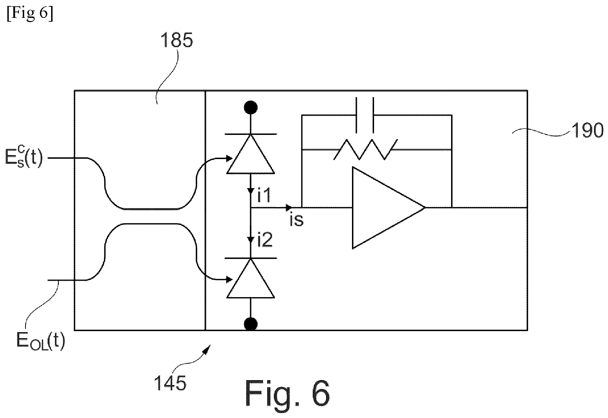

Par ailleurs, afin de générer le battement électromagnétique, le générateur d'ondes 145 peut comporter, comme cela est illustré sur la figure 6, un mélangeur d'ondes 185 et un soustracteur d'ondes 190.

Le mélangeur d'ondes 185 est configuré pour recevoir le rayonnement de sonde collecté $E_s^c(t)$ et l'oscillateur local $E_{OL}(t)$ et pour fournir deux signaux en sortie présentant des intensités respectives $i_1$ et $i_2$ qui sont telles que :

[math 16]

$$\begin{cases} i_1 = i_{sonde} + i_{ol} + 2\sqrt{i_{sonde}.i_{ol}}.\cos\left(2\pi.f\ .t + \varphi\ \right) \\ i_2 = i_{sonde} + i_{ol} - 2\sqrt{i_{sonde}.i_{ol}}.\cos\left(2\pi.f\ .t + \varphi\ \right) \end{cases}$$

Le soustracteur d'onde comporte deux photodiodes montées en opposition afin d'extraire la partie modulée des courants $i_1$ et $i_2$, en obtenant un courant $i_s = i_1 - i_2$ tel que

[math 17]

$$i_s = 4\sqrt{i_{sonde}.i_{ol}}.\cos\left(2\pi.f\ .t + \varphi\ \right)$$

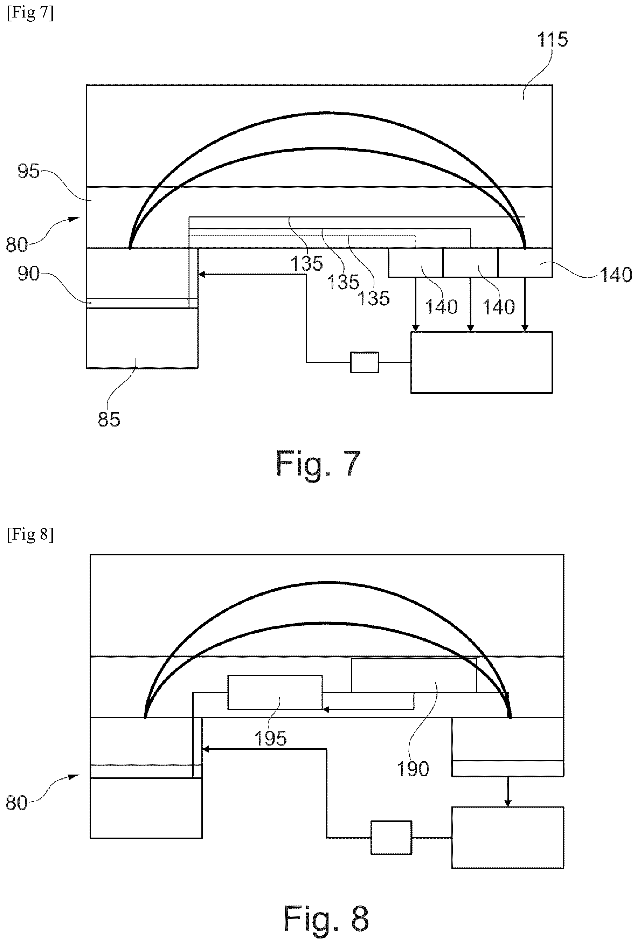

La figure 7 illustre un autre exemple de dispositif selon l'invention. Le dispositif diffère de celui illustré sur la figure 2 en ce que l'unité de détection optique comporte plusieurs récepteurs d'onde 140 et en ce qu'il comporte plusieurs guides d'ondes 135 correspondants.

En sortie du séparateur de faisceau 90, l'oscillateur local peut être guidé séquentiellement vers l'un des récepteurs d'ondes 135. Notamment, la longueur de chaque guide d'onde peut être adaptée pour que la durée de trajet de l'oscillateur local soit différente entre les guides d'ondes. De cette façon, des battements électromagnétiques différents peuvent être générés, induisant différents spectres de densité de puissance et largeurs à mi hauteurs correspondantes, dont l'analyse séquentielle permet d'améliorer l'asservissement du modulateur spatial de lumière.

Par exemple, l'un des guides d'onde peut être configuré pour que la longueur du trajet de l'oscillateur local entre le séparateur de faisceau et l'unité de détection optique correspondante soit égale à la durée du trajet moyen du rayonnement de sonde dans le milieu diffusant entre le séparateur de faisceau et l'unité de détection optique. Un autre des guides d'onde peut être configuré pour que la longueur du trajet de l'oscillateur local entre le séparateur de faisceau et le système de détection optique soit supérieure à la longueur du trajet moyen du rayonnement de sonde à travers le milieu diffusant entre le séparateur de faisceau et l'unité de détection, de façon à maximiser le rapport entre l'intensité du battement électromagnétique sur le bruit du récepteur d'onde.

Ainsi, le dispositif illustré sur la figure 8, le guide d'onde peut être en un matériau présentant un indice optique variant avec la température, choisi pour évoluer d'une manière sensiblement identique à l'indice optique de l'eau. A cette fin, par exemple, le guide d'onde peut être en silicium. Le guide d'onde peut comporter une sonde thermique 190 pour mesurer la température du milieu diffusant, et une unité de régulation thermique 195 pour modifier l'indice optique du guide d'onde, par exemple le en chauffant ou en le refroidissant, à partir de la mesure de la température fournie par la sonde thermique.

Les dispositifs illustrés sur les figures 2 à 8 permettent de focaliser un rayonnement lumineux dans une zone d'étude du milieu diffusant.

Un rayonnement lumineux d'étude peut ensuite être guidé à travers l'unité de modulation optique puis dans le milieu diffusant. Le rayonnement lumineux d'étude est ainsi focalisé dans la zone d'étude grâce à l'exemple de mise en œuvre du procédé de focalisation illustré ci-dessus.

De préférence, le rayonnement lumineux d'étude est guidé à distance du séparateur de faisceau. Autrement dit, le rayonnement lumineux d'étude n'interagit pas avec le séparateur de faisceau. Il peut être généré par la source d'émission lumineuse ou par une source d'émission lumineuse supplémentaire dédiée.

Le rayonnement lumineux d'étude peut présenter une fréquence différente de celle du rayonnement de sonde mise en œuvre dans le procédé de focalisation.

Le rayonnement lumineux d'étude peut être en particulier un rayonnement de sonde spectroscopique.

Notamment, dans l'exemple illustré ci-dessus, le rayonnement de sonde spectroscopique peut présenter une fréquence optique comprise entre $10^{14}$ Hz et $10^{15}$ Hz et être choisi pour détecter et mesurer la teneur en un glucide contenu dans le réseau vasculaire de la zone d'étude. Le rayonnement émis par la zone d'étude peut être collecté par le récepteur d'onde ou par un récepteur d'onde supplémentaire dédié.

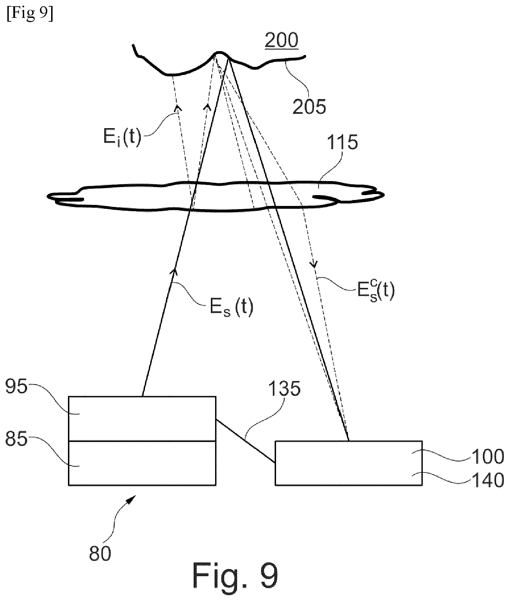

La figure 9 illustre une utilisation d'un exemple de dispositif selon l'invention pour la télédétection d'un objet 200 au sein d'un milieu diffusant 115 comportant des gouttelettes d'un liquide en suspension dans un gaz.

Par exemple, le dispositif selon l'invention est un constituant d'un capteur LIDAR.

Le dispositif comporte les mêmes composants que celui illustré sur les figures 2 à 7. Il peut en différer par une adaptation de la distance entre la source d'émission et l'unité de détection optique en fonction de la distance à laquelle l'objet est recherché.

Dans une première étape, un rayonnement de sonde $E_s(t)$ est émis en direction de l'objet. Les différents faisceaux du rayonnement de sonde $E_i(t)$ sont diffusés sous l'effet des réflexions multiples par les gouttelettes en suspension dans le gaz avant d'atteindre l'objet. Ils sont ensuite réfléchis par la surface 205 de l'objet et sont à nouveau diffusés par le gouttelettes en suspension, jusqu'à ce qu'une partie de ce rayonnement de sonde rétrodiffusé $E_s^c(t)$ soit collecté par l'unité de réception.

Dans une seconde phase, après mélange avec un oscillateur local et détermination de la largeur à mi-hauteur de la densité spectrale de puissance du battement correspondant, l'unité de modulation optique est configurée afin de focaliser le faisceau sur la zone d'étude comportant l'objet.

Le rayonnement lumineux ayant été focalisé sur la zone d'étude, un rayonnement lumineux d'étude peut ensuite être dirigé sur la zone d'étude à travers l'unité de modulation optique. De préférence, le rayonnement lumineux d'étude est aussi émis par l'unité d'émission lumineuse et présente la même fréquence que le rayonnement de sonde.

Avantageusement, la mise en œuvre du procédé de focalisation selon l'invention permet d'améliorer la qualité de la télédétection de l'objet au sein du milieu diffusant. Elle peut avantageusement être mise en œuvre dans un véhicule automobile, notamment autonome, pour la télédétection d'objets, de personnes ou d'autres véhicules dans une brume ou un brouillard.

Bien entendu, l'invention n'est pas limitée aux exemples de réalisation du dispositif ni aux exemples de mise en œuvre du procédé, présentés à titre illustratif et non limitatif. Notamment, dans les exemples illustrés, l'unité de modulation optique est un modulateur spatial de lumière, mais un réseau optique à commande de phase pourrait être mis en œuvre en lieu et place du modulateur spatial de lumière. Par ailleurs, la méthode de spectroscopie peut être mise en œuvre pour vérifier, sur une chaîne de production d'un produit alimentaire, la concentration d'un chromophore contenu dans le produit alimentaire.

**Revendications**

1.  Dispositif (80) pour focaliser un rayonnement lumineux dans une zone d'étude (Ze) au sein d'un milieu diffusant (115), le dispositif comportant :

- une source d'émission lumineuse (85) configurée pour émettre un rayonnement lumineux cohérent présentant au moins une longueur d'onde comprise entre 400 nm et 1700 nm, le rayonnement lumineux étant balayé en fréquence,

- un séparateur de faisceau (90) configuré pour séparer le rayonnement lumineux émis par la source d'émission lumineuse en un rayonnement de sonde $E_s(t)$ et en un oscillateur local $E_{OL}(t)$ respectivement,

- une unité de modulation optique (95) pour modifier l'angle d'incidence sur un milieu diffusant d'au moins un des faisceaux du rayonnement de sonde,

- une unité de détection optique (100) comportant un récepteur d'onde (140) pour collecter l'oscillateur local et au moins une partie du rayonnement de sonde diffusé et réfléchi par le milieu diffusant, dite rayonnement de sonde collecté, et un générateur d'onde (145) configuré pour générer un battement électromagnétique par mélange du rayonnement de sonde collecté et de l'oscillateur local, la source d'émission lumineuse et l'unité de détection optique étant disposées d'un même coté du milieu diffusant, et

- une unité de commande (110) configurée pour asservir l'unité de modulation optique afin de maximiser la valeur $q(f_{moy})$ de la densité d'énergie spectrale de puissance du battement électromagnétique $q(f)$ à la fréquence moyenne du battement électromagnétique $f_{moy}$.

2. Dispositif selon la revendication 1, comportant une unité d'analyse (105) pour déterminer la largeur à mi-hauteur de la densité spectrale de puissance du battement électromagnétique, l'unité de commande (110) étant en outre configurée pour asservir l'unité de modulation optique afin de minimiser la largeur à mi-hauteur $\Delta f$ de la densité spectrale de puissance du battement électromagnétique.

3. Dispositif selon l'une quelconque des revendications 1 et 2, l'unité de modulation optique étant un modulateur spatial de lumière (155) ou un réseau optique à commande de phase.

4. Dispositif selon l'une quelconque des revendications précédentes, le séparateur de faisceau étant configuré pour que l'amplitude de l'oscillateur local soit plus de deux fois, voire plus de cinq fois, voire plus de dix fois, supérieure à l'amplitude du rayonnement de sonde.

5. Dispositif selon l'une quelconque des revendications précédentes, le dispositif comportant un guide d'onde (135) pour conduire l'oscillateur local du séparateur de faisceau jusqu'à l'unité de détection optique, de préférence le matériau constitutif du guide d'onde présentant un indice optique variant avec la température et adapté à la variation de l'indice optique du milieu diffusant avec la température, de préférence le matériau constitutif du guide d'onde étant choisi parmi le silicium, SiN, $SiO_2$ et leurs mélanges.

6. Dispositif selon la revendication précédente, le guide d'onde étant configuré pour que la longueur optique du trajet de l'oscillateur local entre le séparateur de faisceau et l'unité de détection optique soit égale à la longueur optique du trajet moyen du rayonnement de sonde dans le milieu diffusant entre le séparateur de faisceau et l'unité de détection optique.

7. Dispositif selon la revendication 5, le guide d'onde étant configuré pour que la longueur optique du trajet de l'oscillateur local entre le séparateur de faisceau et le système de détection optique soit supérieure à la longueur du trajet moyen du rayonnement de sonde à travers le milieu diffusant entre le séparateur de faisceau et l'unité de détection, de façon à maximiser le rapport entre l'intensité du battement électromagnétique sur le bruit du récepteur d'onde, de préférence le récepteur d'onde comportant une photodiode à jonction PIN, notamment formée de germanium.

8. Dispositif selon l'une quelconque des revendications précédentes, comportant une sonde thermique pour mesurer la température du milieu diffusant et une unité de régulation thermique configurée pour modifier l'indice optique du guide optique en fonction de la température mesurée par la sonde thermique.

9. Dispositif selon l'une quelconque des revendications précédentes, comportant plusieurs guides d'onde pour amener chacun séquentiellement l'oscillateur local du séparateur de faisceau à l'un des récepteurs d'ondes correspondant.

10. Dispositif selon l'une quelconque des revendications précédentes, la détermination de la largeur à mi-hauteur de la densité spectrale de puissance du battement électromagnétique étant obtenue par un traitement comportant une transformée de Fourier du battement électromagnétique.

11. Appareil comportant un dispositif selon l'une quelconque des revendications précédentes, l'appareil étant choisi parmi un appareil de télédétection, notamment de type LIDAR, et un appareil de spectroscopie par réflectance diffuse.

**12.** Procédé de focalisation d'un rayonnement lumineux dans une zone d'étude au sein d'un milieu diffusant au moyen du dispositif selon l'une quelconque des revendications 1 à 11, le procédé comportant :

- l'émission d'un rayonnement lumineux cohérent, le rayonnement lumineux étant balayé en fréquence,
- la séparation du rayonnement lumineux en un oscillateur local et un rayonnement de sonde,
- l'irradiation au moyen du rayonnement de sonde d'une partie du milieu diffusant contenant la zone d'étude,
- la détection d'au moins une partie du rayonnement de sonde diffusé et réfléchi par le milieu diffusant, dite rayonnement de sonde collecté, et de l'oscillateur local,
l'émission du rayonnement lumineux et la détection du rayonnement de sonde collecté étant effectuées d'un même côté du milieu diffusant,
- le mélange du rayonnement de sonde collecté et de l'oscillateur local pour former un battement électromagnétique, et
- la modulation optique du rayonnement de sonde par modification de l'angle d'incidence sur le milieu diffusant d'un ou plusieurs faisceaux du rayonnement de sonde, afin de maximiser la valeur $q(f_{moy})$ de la densité d'énergie spectrale de puissance du battement électromagnétique $q(f)$ à la fréquence moyenne du battement électromagnétique $f_{moy}$.

**13.** Procédé selon la revendication 12, comportant la détermination de la largeur à mi-hauteur $\Delta f$ de la densité spectrale de puissance du battement électromagnétique, la modulation optique du rayonnement de sonde étant de préférence opérée de façon à minimiser la largeur à mi-hauteur de la densité spectrale de puissance du battement électromagnétique.

**14.** Procédé selon l'une quelconque des revendications 12 et 13, le milieu diffusant étant un tissu biologique, notamment une partie d'un corps, en particulier humain, ou le milieu diffusant étant une suspension de gouttelettes d'un liquide, notamment d'eau, dans un gaz, notamment l'air.

**15.** Méthode de spectroscopie par réflectance diffuse, la méthode comportant :

- la mise en œuvre du procédé de focalisation selon la revendication 14, la zone d'étude comportant des chromophores,
- l'émission d'un rayonnement de sonde spectroscopique vers l'unité de modulation optique afin de focaliser le rayonnement de sonde spectroscopique sur la zone d'intérêt, et
- la détection de la partie du rayonnement réémis par les chromophores sensibles au rayonnement de sonde spectroscopique, qui est diffusée et réfléchie par le milieu diffusant.

**16.** Méthode de télédétection d'un objet (200) dans une suspension, dans un gaz, de gouttelettes d'un liquide, la méthode comportant :

- la mise en œuvre du procédé de focalisation selon la revendication 14, la zone d'étude contenant l'objet,
- l'émission d'un rayonnement de sonde de télédétection vers l'unité de modulation optique afin de focaliser le rayonnement de sonde de télédétection dans la zone d'intérêt, et
- la détection de la partie du rayonnement réémis par l'objet qui est diffusée et réfléchie par le milieu diffusant.

**Patentansprüche**

**1.** Vorrichtung (80) zur Fokussierung einer Lichtstrahlung in einem Untersuchungsbereich (Ze) innerhalb eines streuenden Milieus (115), wobei die Vorrichtung umfasst:

- eine Lichtemissionsquelle (85), die dazu ausgestaltet ist, eine kohärente Lichtstrahlung zu emittieren, die mindestens eine Wellenlänge zwischen 400 nm und 1700 nm aufweist, wobei die Lichtstrahlung frequenzgewobbelt wird,
- einen Strahlteiler (90), der dazu ausgestaltet ist, die von der Lichtemissionsquelle emittierte Lichtstrahlung in eine Sondenstrahlung $E_s(t)$ und in einen Lokaloszillator $E_{OL}(t)$ zu teilen,
- eine optische Modulationseinheit (95), um den Einfallswinkel an einem streuenden Milieu mindestens eines der Strahlen der Sondenstrahlung zu verändern,

---

**EP 4 197 433 B1**

- eine optische Detektionseinheit (100), die einen Wellenempfänger (140) umfasst, um den Lokaloszillator und mindestens einen Teil der von dem streuenden Milieu gestreuten und reflektierten Sondenstrahlung, gesammelte Sondenstrahlung genannt, zu sammeln, und einen Wellenerzeuger (145), der dazu ausgestaltet ist, eine elektromagnetische Überlagerung durch Mischen der gesammelten Sondenstrahlung und des Lokaloszillators zu erzeugen, wobei die Lichtemissionsquelle und die optische Detektionseinheit auf einer selben Seite des streuenden Milieus angeordnet sind, und

- eine Steuerungseinheit (110), die dazu ausgestaltet ist, die optische Modulationseinheit zu regeln, um den Wert $q(f_{moy})$ der spektralen Leistungsenergiedichte der elektromagnetischen Überlagerung $q(f)$ bei der mittleren Frequenz der elektromagnetischen Überlagerung $f_{moy}$ zu maximieren.

2. Vorrichtung nach Anspruch 1, umfassend eine Analyseeinheit (105), um die Breite auf halber Höhe der spektralen Leistungsdichte der elektromagnetischen Überlagerung zu bestimmen, wobei die Steuerungseinheit (110) ferner dazu ausgestaltet ist, die optische Modulationseinheit zu regeln, um die Breite auf halber Höhe $\Delta f$ der spektralen Leistungsdichte der elektromagnetischen Überlagerung zu minimieren.

3. Vorrichtung nach einem der Ansprüche 1 und 2, wobei die optische Modulationseinheit ein räumlicher Lichtmodulator (155) oder ein phasengesteuertes optisches Array ist.

4. Vorrichtung nach einem der vorhergehenden Ansprüche, wobei der Strahlteiler dazu ausgestaltet ist, dass die Amplitude des Lokaloszillators mehr als zwei Mal oder sogar mehr als fünf Mal oder sogar mehr als zehn Mal größer als die Amplitude der Sondenstrahlung ist.

5. Vorrichtung nach einem der vorhergehenden Ansprüche, wobei die Vorrichtung einen Wellenleiter (135) umfasst, um den Lokaloszillator von dem Strahlteiler bis zu der optischen Detektionseinheit zu führen, wobei das Material, aus dem der Wellenleiter besteht, bevorzugt einen sich mit der Temperatur ändernden optischen Index aufweist und an die Änderung des optischen Index des streuenden Milieus mit der Temperatur angepasst ist, wobei das Material, aus dem der Wellenleiter besteht, bevorzugt aus Silicium, SiN, $SiO_2$ und ihren Mischungen ausgewählt ist.

6. Vorrichtung nach dem vorhergehenden Anspruch, wobei der Wellenleiter dazu ausgestaltet ist, dass die optische Länge des Wegs des Lokaloszillators zwischen dem Strahlteiler und der optischen Detektionseinheit gleich der optischen Länge des mittleren Wegs der Sondenstrahlung in dem streuenden Milieu zwischen dem Strahlteiler und der optischen Detektionseinheit ist.

7. Vorrichtung nach Anspruch 5, wobei der Wellenleiter dazu ausgestaltet ist, dass die optische Länge des Wegs des Lokaloszillators zwischen dem Strahlteiler und dem optischen Detektionssystem größer als die Länge des mittleren Wegs der Sondenstrahlung durch das streuende Milieu hindurch zwischen dem Strahlteiler und der Detektionseinheit ist, so dass das Verhältnis der Intensität der elektromagnetischen Überlagerung zum Rauschen des Wellenempfängers maximiert ist, wobei der Wellenempfänger bevorzugt eine Photodiode mit PIN-Übergang umfasst, die insbesondere aus Germanium gebildet ist.

8. Vorrichtung nach einem der vorhergehenden Ansprüche, umfassend eine thermische Sonde zum Messen der Temperatur des streuenden Milieus und eine thermische Regelungseinheit, die dazu ausgestaltet ist, den optischen Index des Lichtleiters in Abhängigkeit von der von der thermischen Sonde gemessenen Temperatur zu verändern.

9. Vorrichtung nach einem der vorhergehenden Ansprüche, umfassend mehrere Wellenleiter, um jeweils sequenziell den Lokaloszillator von dem Strahlteiler zu einem der entsprechenden Wellenempfänger zu führen.

10. Vorrichtung nach einem der vorhergehenden Ansprüche, wobei das Bestimmen der Breite auf halber Höhe der spektralen Leistungsdichte der elektromagnetischen Überlagerung durch eine Verarbeitung erhalten wird, die eine Fourier-Transformation der elektromagnetischen Überlagerung umfasst.

11. Gerät, umfassend eine Vorrichtung nach einem der vorhergehenden Ansprüche, wobei das Gerät aus einem Ferndetektionsgerät, insbesondere vom Typ LIDAR, und einem Gerät zur streuenden Reflexionsspektroskopie ausgewählt ist.

12. Verfahren zur Fokussierung einer Lichtstrahlung in einem Untersuchungsbereich innerhalb eines streuenden Milieus mittels der Vorrichtung nach einem der Ansprüche 1 bis 11, wobei das Verfahren umfasst:

**EP 4 197 433 B1**

- das Emittieren einer kohärenten Lichtstrahlung, wobei die Lichtstrahlung frequenzgewobbelt wird,
- das Teilen der Lichtstrahlung in einen Lokaloszillator und eine Sondenstrahlung,
- das Bestrahlen eines Teils des den Untersuchungsbereich enthaltenden streuenden Milieus mittels der Sondenstrahlung,
- das Detektieren mindestens eines Teils der von dem streuenden Milieu gestreuten und reflektierten Sondenstrahlung, gesammelte Sondenstrahlung genannt, und des Lokaloszillators,
wobei das Emittieren der Lichtstrahlung und das Detektieren der gesammelten Sondenstrahlung auf einer selben Seite des streuenden Milieus erfolgen,
- das Mischen der gesammelten Sondenstrahlung und des Lokaloszillators, um eine elektromagnetische Überlagerung zu bilden, und
- das optische Modulieren der Sondenstrahlung durch Verändern des Einfallswinkels an dem streuenden Milieu eines oder mehrerer Strahlen der Sondenstrahlung, um den Wert $q(f_{moy})$ der spektralen Leistungsenergiedichte der elektromagnetischen Überlagerung $q(f)$ bei der mittleren Frequenz der elektromagnetischen Überlagerung $f_{moy}$ zu maximieren.

13. Verfahren nach Anspruch 12, umfassend das Bestimmen der Breite auf halber Höhe $\Delta f$ der spektralen Leistungsdichte der elektromagnetischen Überlagerung, wobei das optische Modulieren der Sondenstrahlung bevorzugt so durchgeführt wird, dass die Breite auf halber Höhe der spektralen Leistungsdichte der elektromagnetischen Überlagerung minimiert wird.

14. Verfahren nach einem der Ansprüche 12 und 13, wobei das streuende Milieu ein biologisches Gewebe, insbesondere ein Teil eines, insbesondere menschlichen, Körpers ist, oder wobei das streuende Milieu eine Suspension von Tröpfchen einer Flüssigkeit, insbesondere Wasser, in einem Gas, insbesondere Luft, ist.

15. Methode zur streuenden Reflexionsspektroskopie, wobei die Methode umfasst:

- das Durchführen des Verfahrens zur Fokussierung nach Anspruch 14, wobei der Untersuchungsbereich Chromophore umfasst,
- das Emittieren einer spektroskopischen Sondenstrahlung zu der optischen Modulationseinheit hin, um die spektroskopische Sondenstrahlung auf den Bereich von Interesse zu fokussieren, und
- das Detektieren des Teils der von den für die spektroskopische Sondenstrahlung empfindlichen Chromophoren reemittierten Strahlung, der von dem streuenden Milieu gestreut und reflektiert wird.

16. Methode zur Ferndetektion eines Objekts (200) in einer Suspension, in einem Gas, von Tröpfchen einer Flüssigkeit, wobei die Methode umfasst:

- das Durchführen des Verfahrens zur Fokussierung nach Anspruch 14, wobei der Untersuchungsbereich das Objekt enthält,
- das Emittieren einer Ferndetektionssondenstrahlung zu der optischen Modulationseinheit hin, um die Ferndetektionssondenstrahlung in dem Bereich von Interesse zu fokussieren, und
- das Detektieren des Teils der von dem Objekt reemittierten Strahlung, der von dem streuenden Milieu gestreut und reflektiert wird.

**Claims**

1. Device (80) for focusing light radiation into a study region (Ze) within a scattering medium (115), the device comprising:

- a light-emitting source (85) configured to emit coherent light radiation having at least one wavelength between 400 nm and 1700 nm, the light radiation being scanned in frequency,
- a beam splitter (90) configured to split the light radiation emitted by the light-emitting source into probe radiation $E_s(t)$ and a local oscillator $E_{OL}(t)$, respectively,
- an optical modulating unit (95) for modifying the angle of incidence on a scattering medium of at least one of the beams of probe radiation,
- an optical detecting unit (100) comprising a wave receiver (140) for collecting the local oscillator and at least a portion of the probe radiation scattered and reflected by the scattering medium, which is referred to as the collected probe radiation, and a wave generator (145) configured to generate an electromagnetic beat by mixing the collected probe radiation and the local oscillator, the light-emitting source and the optical detecting unit being

**16**

placed on the same side of the scattering medium, and

- a control unit (110) configured to automatically control the optical modulating unit in order to maximize the value $q(f_{moy})$ of the power spectral energy density of the electromagnetic beat $q(f)$ at the average frequency $f_{moy}$ of the electromagnetic beat.

2. Device according to Claim 1, comprising an analysing unit (105) for determining the full width at half maximum of the power spectral density of the electromagnetic beat, the control unit (110) further being configured to automatically control the optical modulating unit in order to minimize the full width at half maximum $\Delta f$ of the power spectral density of the electromagnetic beat.

3. Device according to either of Claims 1 and 2, the optical modulating unit being a spatial light modulator (155) or an optical phased array.

4. Device according to any of the preceding claims, wherein the beam splitter is configured so that the amplitude of the local oscillator is more than twice, or more than five times, or even more than ten times, greater than the amplitude of the probe radiation.

5. Device according to any of the preceding claims, the device comprising a waveguide (135) for conveying the local oscillator from the beam splitter to the optical detecting unit, the material from which the waveguide is made preferably having a refractive index that varies with temperature and that matches the variation in the refractive index of the scattering medium with temperature, the material from which the waveguide is made preferably being chosen from silicon, SiN, $SiO_2$ and mixtures thereof.

6. Device according to the preceding claim, the waveguide being configured so that the optical length of the path of the local oscillator between the beam splitter and the optical detecting unit is equal to the optical length of the average path of the probe radiation in the scattering medium between the beam splitter and the optical detecting unit.

7. Device according to Claim 5, the waveguide being configured so that the optical length of the path of the local oscillator between the beam splitter and the optical detection system is greater than the average path length of the probe radiation through the scattering medium between the beam splitter and the detecting unit, so as to maximize the ratio between the amplitude of the electromagnetic beat and the noise of the wave receiver, the wave receiver preferably comprising a PIN photodiode, in particular one formed from germanium.

8. Device according to any of the preceding claims, comprising a temperature probe for measuring the temperature of the scattering medium and a temperature-regulating unit configured to modify the refractive index of the optical guide depending on the temperature measured by the temperature probe.

9. Device according to any of the preceding claims, comprising a plurality of waveguides each for sequentially feeding the local oscillator of the beam splitter to a corresponding one of the wave receivers.

10. Device according to any of the preceding claims, the full width at half maximum of the power spectral density of the electromagnetic beat being determined by processing comprising a Fourier transform of the electromagnetic beat.

11. Apparatus comprising a device according to any of the preceding claims, the apparatus being chosen from a remote-sensing apparatus, in particular of LIDAR type, and a diffuse-reflectance-spectroscopy apparatus.

12. Method for focusing light radiation into a study region within a scattering medium by means of the device according to any of Claims 1 to 11, the method comprising:

- emitting coherent light radiation, the light radiation being scanned in frequency,
- splitting the light radiation into a local oscillator and probe radiation,
- irradiating by means of the probe radiation a portion of the scattering medium containing the study region,
- detecting at least a portion of the probe radiation scattered and reflected by the scattering medium, which is referred to as the collected probe radiation, and the local oscillator,
the light radiation being emitted and the collected probe radiation being detected on the same side of the scattering medium,
- mixing the collected probe radiation and the local oscillator to form an electromagnetic beat, and
- performing optical modulation of the probe radiation by modifying the angle of incidence on the scattering medium of one or more beams of probe radiation, in order to maximize the value $q(f_{moy})$ of the power spectral energy density of the electromagnetic beat $q(f)$ at the average frequency $f_{moy}$ of the electromagnetic beat.

13. Method according to Claim 12, comprising determining the full width at half maximum $\Delta f$ of the power spectral density of the electromagnetic beat, the optical modulation of the probe radiation preferably being performed so as to minimize the full width at half maximum of the power spectral density of the electromagnetic beat.

14. Method according to either of Claims 12 and 13, the scattering medium being a biological tissue, in particular a part of a body, particularly a human body, or the scattering medium being a suspension of droplets of a liquid, in particular water, in a gas, in particular air.

15. Diffuse-reflectance-spectroscopy procedure, the procedure comprising:

   - implementing the focusing method according to Claim 14, the study region containing chromophores,
   - emitting spectroscopic probe radiation toward the optical modulating unit in order to focus the spectroscopic probe radiation onto the region of interest, and
   - detecting the portion of the radiation re-emitted by chromophores sensitive to the spectroscopic probe radiation, that is scattered and reflected by the scattering medium.

16. Procedure for remotely sensing an object (200) in a suspension, in a gas, of droplets of a liquid, the procedure comprising:

   - implementing the focusing method according to Claim 14, the study region containing the object,
   - emitting remote-sensing probe radiation toward the optical modulating unit in order to focus the remote-sensing probe radiation into the region of interest, and
   - detecting the portion of the radiation re-emitted by the object that is scattered and reflected by the scattering medium.

[Fig 1]

Fig. 1

[Fig 2]

Fig. 2

[Fig 3]

Fig. 3

[Fig 4]

Fig. 4

[Fig 5]

Fig. 5

[Fig 6]

Fig. 6

[Fig 7]

Fig. 7

[Fig 8]

Fig. 8

[Fig 9]

$E_i(t)$

$E_s(t)$

$E_s^c(t)$

Fig. 9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 6958815 B2
- US 20130274612 A1
- WO 2020201156 A1
- KR 20210051683 A

### Littérature non-brevet citée dans la description

- **PETITDIDIER, N. A.** Contact, high-resolution spatial diffuse reflectance imaging system for skin condition diagnosis. *Journal of Biomedical Optics*, 2018, vol. 23 (11)
- **KOENIG, A. N.** Contact, High-Resolution Spatial Diffuse Reflectance Imaging System for Skin Condition Diagnosis: A First-in-Human Clinical trial. *Journal of Biomedical Optics*, 2021, vol. 26 (01)
- **BYKOV et al.** Time gating in glucose sensing with ultrashort pulses. Advanced Laser Technologies.. *Proc. SPIE 7022, Advanced Laser Technologies*, 2007
- **VELLEKOOP et al.** Focusing coherent light through opaque stronglys cattering media. *Optics Express*, 2007, vol. 32 (16), 2309-2311
- **JAEDUCK JANG et al.** Complex wavefront shapingfor optimal depth-selective focusing in optical coherence tomography. *Optics Express*, 30 January 2013, vol. 21 (3), 2890-2902
- **A. BRIMONT et al.** Strong electro-optical modulation enhancement in a slow wave corrugated waveguide. *Optics Express*, 2009, vol. 17 (11), 9204-9211